(19) Europäisches Patentamt / European Patent Office / Office européen des brevets

(11) **EP 4 202 855 A1**

(12) **EUROPEAN PATENT APPLICATION**

(43) Date of publication:
**28.06.2023 Bulletin 2023/26**

(21) Application number: **21306909.9**

(22) Date of filing: **22.12.2021**

(51) International Patent Classification (IPC):
*G06V 10/774* (2022.01) *G06V 10/82* (2022.01)
*A61B 6/00* (2006.01) *G16H 30/40* (2018.01)
*G16H 50/50* (2018.01) *G16H 50/70* (2018.01)
*G01R 33/56* (2006.01)

(52) Cooperative Patent Classification (CPC):
**A61B 6/481; A61B 6/52; G01R 33/5601;**
**G01R 33/5608; G06V 10/774; G06V 10/82;**
**G16H 30/20; G16H 30/40; G16H 50/20;**
**G16H 50/50; G16H 50/70;** G06V 2201/031

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Guerbet**
  **93420 Villepinte (FR)**
• **Institut Gustave-Roussy**
  **94800 Villejuif (FR)**

(72) Inventors:
• **BôNE, ALEXANDRE**
  **75009 PARIS (FR)**
• **ROHé, Marc-Michel**
  **92120 MONTROUGE (FR)**
• **LASSAU, Nathalie**
  **94160 SAINT-MANDE (FR)**
• **AMMARI, Samy**
  **94700 MAISONS-ALFORT (FR)**

(74) Representative: **Regimbeau**
**20, rue de Chazelles**
**75847 Paris Cedex 17 (FR)**

(54) **METHOD FOR PROCESSING AT LEAST A PRE-CONTRAST IMAGE AND A CONTRAST IMAGE RESPECTIVELY DEPICTING A BODY PART PRIOR TO AND AFTER AN INJECTION OF A DOSE OF CONTRAST AGENT**

(57) The present invention relates to method for medical imaging, the method being characterized in that it comprises the implementation, by a data processor (11b) of a second server (1b), of steps of:

(a) Obtaining at least a candidate pre-contrast image and a candidate contrast image respectively depicting a body part prior to and after an injection of a first dose of contrast agent, wherein said first dose is higher than a predetermined low dose, the predetermined reduced dose being lower than a predetermined standard dose; a convolutional neural network, CNN, being trained to reconstruct, from at least a training pre-contrast image and a training contrast image respectively depicting a body part prior to and after an injection of said low dose of contrast agent, a training contrast image depicting said body part after an injection of said standard dose of contrast agent;

(b) Applying the CNN to the candidate pre-contrast image and the candidate contrast image, so as to generate a synthetic contrast image simulating said body part after an injection of a second dose of contrast agent which is higher than the standard dose.

FIG. 2

**Description**

FIELD OF THE INVENTION

The field of this invention is that of machine/deep learning.

More particularly, the invention relates to methods for processing at least a pre-contrast image and a contrast image respectively depicting a body part prior to and after an injection of a first standard dose of contrast agent, in particular for generating a synthetic contrast image simulating said body part after an injection of a second dose of contrast agent which is higher than the standard dose.

BACKGROUND OF THE INVENTION

Contrast agents are substances used to increase the contrast of structures or fluids within the body in medical imaging.

They usually absorb or alter external radiations emitted by the medical imaging device. In x-rays, contrast agents enhance the radiodensity in a target tissue or structure. In MRIs, contrast agents modify the relaxation times of nuclei within body tissues in order to alter the contrast in the image.

Contrast agents are commonly used to improve the visibility of lesions, notably in neuroimaging for the initial diagnosis and treatment planning of brain tumors, such as glioma, brain metastasis, meningioma.

Dynamic susceptibility contrast (DSC) and Dynamic contrast enhanced (DCE), respectively leveraging T2 and T1 effects, are the two most common techniques for MRI. In both cases, a gadolinium-based contrast agent (GBCA) is injected intravenously to the patient and rapid repeated imaging is performed in order to obtain a temporal sequence of images.

GBCA injection increases the sensitivity of MRI, allowing for instance the identification of smaller carcinogenic nodules, an earlier treatment initiation and, in turn, improving patient survival and quality of life. One solution to further increase MRI sensitivity is to increase the injected quantity of GBCA.

However, based on precautionary considerations, recent clinical guidelines suggest using the minimum dosage that achieves a sufficient contrast enhancement, , and GBCA usage should therefore be as parsimonious as possible.

Three complementary research avenues for nevertheless improving MRI contrast and its lesion detection performance can be identified:

- Firstly, new contrast agents with improved chemical properties have the potential to improve the image contrast without increasing the injected dose of gadolinium. However, such agents are still under testing, and are likely to be even more expensive than GBCA.
- Secondly, novel MRI sequences, with or without contrast agent, may respectively replace or complement the routine contrast-enhanced T1 sequences. In particular, the turbo spin echo T1 sequence with variable flip angles (TSE) proved more sensitive than its gradient echo (GRE) counterpart and is now recommended for brain tumor imaging, although some qualitative limitations were also identified in addition to the longer minimum scan time that may favor motion artifacts.
- Third, promising deep learning approaches are being increasingly proposed to automatically detect and delineate tumors, bearing the promise of uniform and constant accuracy levels with virtually instantaneous reading times. However, these deep learning algorithms are still under research and their performance remains insufficient for immediate large-scale deployment.

There is consequently still a need for a new method to further improve sensitivity for contrast enhanced medical imaging.

SUMMARY OF THE INVENTION

For these purposes, the present invention provides a method for medical imaging, the method being characterized in that it comprises the implementation, by a data processor of a second server, of steps of:

(a) Obtaining at least a candidate pre-contrast image and a candidate contrast image respectively depicting a body part prior to and after an injection of a first dose of contrast agent, wherein said first dose is higher than a predetermined low dose, the predetermined reduced dose being lower than a predetermined standard dose; a convolutional neural network, (CNN), being trained to reconstruct, from at least a training pre-contrast image and a training contrast image respectively depicting a body part prior to and after an injection of said low dose of contrast agent, a training contrast image depicting said body part after an injection of said standard dose of contrast agent;

(b) Applying the CNN to the candidate pre-contrast image and the candidate contrast image, so as to generate a synthetic contrast image simulating said body part after an injection of a second dose of contrast agent which is higher than the standard dose.

Preferred but non limiting features of the present invention are as it follows:

Said low dose is between 10 and 50% of the standard dose.
Said low dose is between 1/5 and 1/3 of the standard dose, preferably around 25%
Said first dose is at least 50% of the standard dose.
Said first dose is at least 80% of the standard dose, preferably around 100% of the standard dose

So that said second dose is at least 240% of the standard dose, preferably between 320% and 400% of the standard dose.

The candidate pre-contrast image and the candidate contrast image are acquired by a medical imaging device connected to the second server, in particular an MRI scanner.

Said at least one pre-contrast image and contrast image are T1-weighted images.

Step (a) comprises obtaining three candidate pre-contrast images, including the T1-weighted pre-contrast image, a T2-flair-weighted pre-contrast image, and an ADC pre-contrast map; the CNN being trained to reconstruct, from a training T1-weighted pre-contrast image, a training T2-flair-weighted pre-contrast image, and a training ADC pre-contrast map and a training T1-weighted contrast image respectively depicting a body part prior to and after an injection of said low dose of contrast agent, a training T1-weighted contrast image depicting said body part after an injection of said standard dose of contrast agent; step (b) comprising applying the CNN to the three candidate pre-contrast image and the candidate contrast image, so as to generate a synthetic T1-weighted contrast image simulating said body part after an injection of a second dose of contrast agent which is higher than the standard dose.

Said CNN comprises an encoder branch and then a decoder branch, with skip connections between the encoder branch and decoder branch.

The method comprises a previous step of training, by a data processor of a first server, said CNN from a base of sequences of a training pre-contrast image, a first training contrast image and a second training image respectively depicting a body part prior to, after an injection of said low dose of contrast agent, and after an injection of said standard dose of contrast agent.

According to a second and a third aspect, the invention provides a computer program product comprising code instructions to execute a method according to the first aspect for medical imaging; and a computer-readable medium, on which is stored a computer program product comprising code instructions for executing said method according to the first aspect for medical imaging.

BRIEF DESCRIPTION OF THE DRAWINGS

The above and other objects, features and advantages of this invention will be apparent in the following detailed description of an illustrative embodiment thereof, which is to be read in connection with the accompanying drawings wherein:

- figure 1 illustrates an example of architecture in which the method according to the invention is performed;
- figure 2 represents an example of training pipeline and operation pipeline of a CNN in the method according to the invention;
- figure 3 represents a preferred architecture of CNN used in the method according to the invention;
- figure 4 illustrates an embodiment of the method according to the invention;
- figure 5 compares T1 c and T1 c+ contrast images obtained using the method according to the invention.

DETAILED DESCRIPTION OF A PREFERRED EMBODIMENT

*Architecture*

The present invention proposes a method for medical imaging, in particular for processing at least a candidate pre-contrast image and a candidate contrast image respectively depicting a body part prior to and during or after an injection of a first dose of contrast agent, a convolutional neural network (CNN).

By pre-contrast image, or "plain" image, it is meant an image depicting a given body part (to be monitored) prior to an injection of contrast agent, for a person or an animal. By contrast image it is meant an image depicting said body part during or after the injection of contrast agent.

EP 4 202 855 A1

In other words, if there is a temporal sequence of images, the first one is the pre-contrast image, and each of the following is a contrast image. Note that the contrast images may be images of a given phase (e.g. arterial, portal, delayed) or fully dynamic contrast enhanced (DCE).

In the following description, we will take the preferred example of brain imaging, i.e. said body part is the brain.

The (pre-contrast or contrast) images are either directly acquired, or derived from images directly acquired, by a medical imaging device of the scanner type.

Said imaging with injection of contrast agent may be:

- CT (Computed Tomography) → the medical imaging device is an X-ray rotational scanner capable of tomographic reconstruction;
- MRI (Magnetic Resonance Imaging) → the medical imaging device is an MRI scanner;
- Mammography → the medical imaging device is an X-ray mammograph;
- Etc.

The acquisition of a said image may involve the injection of a contrast agent such as gadolinium (GBCA) for MRI or appropriate x-ray contrast agents.

Note that the "images" can be 2D objects (with two spatial dimensions) but also possibly 3D objects (with three spatial dimensions), i.e. volumes constituted of stacks of bidimensional images as "slices" according to a third spatial dimension - in other words, we have 2+1 spatial dimensions).

Furthermore, we can have a plurality of pre-contrast images and/or candidate contrast images.

In a preferred MRI embodiment, we have at least a T1-weighted pre-contrast image and a T1-weighted contrast image, in particular GRE (gradient echo) T1-weighted pre-contrast/contrast images, but we may further have:

- a T2-flair-weighted pre-contrast image (in particular TSE (turbospin echo)); and/or
- an ADC (apparent diffusion coefficient) pre-contrast map (automatically converted from EPI (echo-planar) DWI (diffusion weighted)),

Note that a TSE (turbospin echo) T1-weighted contrast image can also be available, but it will not be presently used.

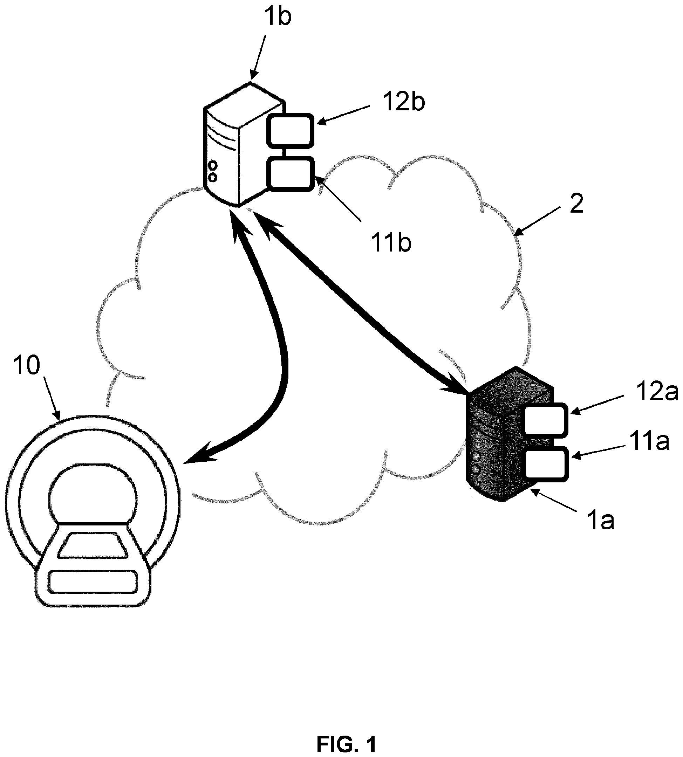

The above-mentioned methods are implemented within an architecture such as illustrated in **Figure 1**, by means of a first and/or second server 1a, 1b. The first server 1a is the training server (implementing a training method of the CNN) and the second server 1b is a processing server (implementing the processing method). It is fully possible that these two servers may be merged.

Each of these servers 1a, 1b is typically remote computer equipment connected to an extended network 2 such as the Internet for data exchange. Each one comprises data processing means 11a, 11b of processor type (in particular the data processor 11a of the first server 1a have strong computing power, since learning is long and complex compared with ordinary use of the trained models), and optionally storage means 12a, 12b such as a computer memory e.g. a hard disk. The second server 1b may be connected to one or more medical imaging devices 10 as client equipment, for providing images to be processed, and receiving back parameters.

Note that it is supposed that the imaging device 10 comprises an injector for performing the injection of contrast agent, said injector applying the injection parameters.

The memory 12a of the first server 1a stores a training database i.e. a set of images referred to as training images (as opposed to so-called inputted images that precisely are sought to be processed). Each image of the database could be pre-contrast or contrast, and contrast images may be labelled in terms of a dose of contrast agent injected. Note that images corresponding the same injection (i.e. forming a temporal sequence) are associated into sequences.

*Dose minimization CNN*

In the following description, we will refer to a "low dose" and a "standard dose" of injected contrast agent. Both are predetermined. For convenience, a contrast image depicting a body part after an injection of the standard dose of contrast agent will be referred to as "standard dose contrast image", and a contrast image depicting a body part after an injection of the low dose of contrast agent will be referred to as "low dose contrast image".

The standard dose, or "full-dose", is the recommended dose which is generally used for a medical examination. While side effects are possible, such dose is not particularly dangerous for the patient, and allows an image quality level sufficient for analysis/diagnostic purposes. In the case of GBCA, the standard dose is 0.1mmol/kg.

The low dose, or "reduced dose", is a dose which is lower than the standard dose, and which causes less effects to the patient health. Said low dose may be any fraction of the standard dose and is preferably between 1/10 and 1/2 (between 10 and 50%) of the standard dose, preferably between 1/5 and 1/3 (between 20 and 33%) of the

4

standard dose, preferably around 1/4 (25%). In the case of GBCA, the low dose is for instance 0.025mmol/kg (25% of 0.1 mmol/kg).

Obviously, the low dose contrast image presents less contrast than the standard dose contrast image. And naively amplifying the contrast enhancement of a x% low-dose CE-MRI by a factor of 100/x results in poor image quality with widespread noise and ambiguous structures.

The present CNN is trained to reconstruct, from at least a training pre-contrast image and a training contrast image respectively depicting a body part prior to and after an injection of said low dose of contrast agent, a training contrast image depicting said body part after an injection of said standard dose of contrast agent.

Such CNNs are known to the skilled person, see for instance the application WO2019074938 or the document Ammari S, Bône A, Balleyguier C, et al. Can Deep Learning Replace Gadolinium in Neuro-Oncology? Invest Radiol. 2021; Publish Ah(00):1-9. doi: 10.10971rli.00000000000008, and allow to reduce the contrast agent dose: by applying the CNN to a candidate pre-contrast image and a candidate contrast image depicting a body part prior to and after an injection of only the low dose of contrast agent, a synthetic contrast image simulating said body part after an injection of the standard dose can be generated.

To rephrase, this CNN is able to predict the standard dose contrast image from the pre-contrast image and the low dose contrast image, and hence can be referred as "dose minimization CNN".

Indeed, we can still have the quality of the standard dose contrast image while only injecting the low dose of contrast agent.

Possible embodiments of this CNN and training methods will be described below.

Preferably, said CNN use as inputs as least the T1-weighted pre-contrast/contrast images (and possibly also the T2-flair-weigthed pre-contrast image and/or the ADC pre-contrast map) and outputs another T1-weighted contrast image.

The present method proposes a clever use of this dose minimization CNN to virtually increase the dose, by applying the dose reduction CNN on candidate contrast image depicting a body part after an injection of a "first dose" higher than low dose of contrast agent, possibly up to the standard dose (but not above). This leads to the generation of a synthetic contrast image simulating said body part after an injection of a "second dose" of contrast agent which is higher than the standard dose, i.e. a "super dose".

Consequently, we are able to simulate the injection of a high dose that could be dangerous to the patient but allows a very high quality of image, while only injecting no more than the standard dose of contrast agent.

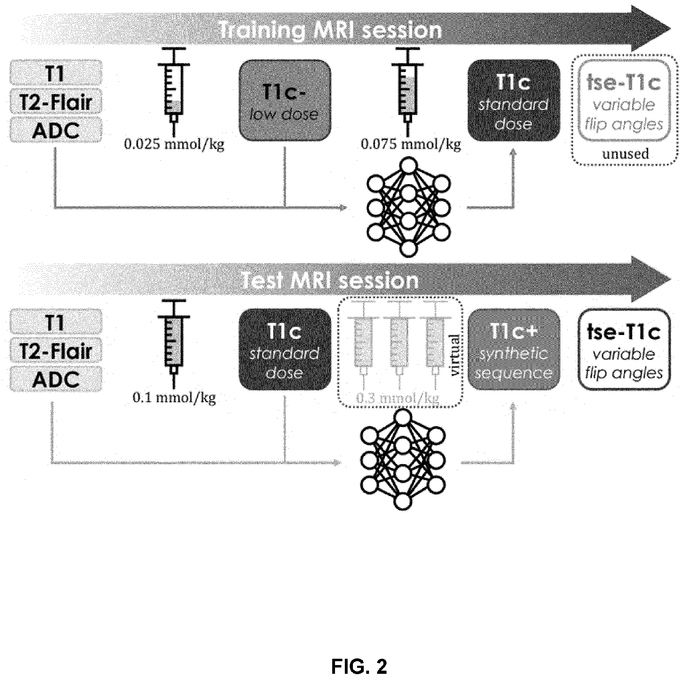

In other words, the CNN is not used for the task it is actually trained for, which is very unusual, see the two exemplary pipelines of **figure 2** wherein the standard dose contrast image is directly used as the first dose contrast image.

In this figure, the T1-weighted pre-contrast image is referred to as "T1", the T2-flair-weighted pre-contrast image is referred to as "T2", the ADC pre-contrast map is referred to as "ADC", the low-dose T1-weighted contrast image is referred to as "T1c-", the standard-dose T1-weighted contrast image is referred to as "T1c", the "super dose" T1-weighted contrast image is referred to as "T1c+".

Hypothesizing that the CNN primarily learned to amplify the difference of contrast between their pre-contrast and post-contrast inputs, replacing T1c- by T1c images at inference was expected to synthesize approximate quadruple-dose T1c+ images.

For instance, assuming the low dose ($D_{c-}$) is x% of the standard dose (Dc):

$$D_{c-} = x/100 * D_c$$

Because the same CNN is used, the first and second doses ($D_1$, $D_2$) can be written with respect to the same equation: $D_1 = x/100 * D_2$.

And thus: $D_{c-} < D_1 \leq D_c$

$$x/100 * D_c < x/100 * D_2 \leq D_c$$

$D_c < D_2 \leq 100/x * D_c$, with $D_{c+} = 100/x * D_c$

Consequently, in the embodiment wherein the low dose is 25% of the standard dose, the second dose can reach 400% of the standard dose if the standard dose is used as first dose (see figure 2). In other words, if the standard dose is 0.1 mmol.kg, the second dose can reach 0.4 mmol/kg (virtual supplementary injection of 0.3 mmol/kg) hence the name "super dose".

Note that said first dose is advantageously at least 80% of the standard dose, preferably around 100% of the standard dose, meaning that the second dose is typically at least 160% of the standard dose (80%/50%), preferably at least 240% (80%/33%), and preferably between 320% and 400% of the standard dose (80%/25% and 100%/25%).

*Tridimensional UNet*

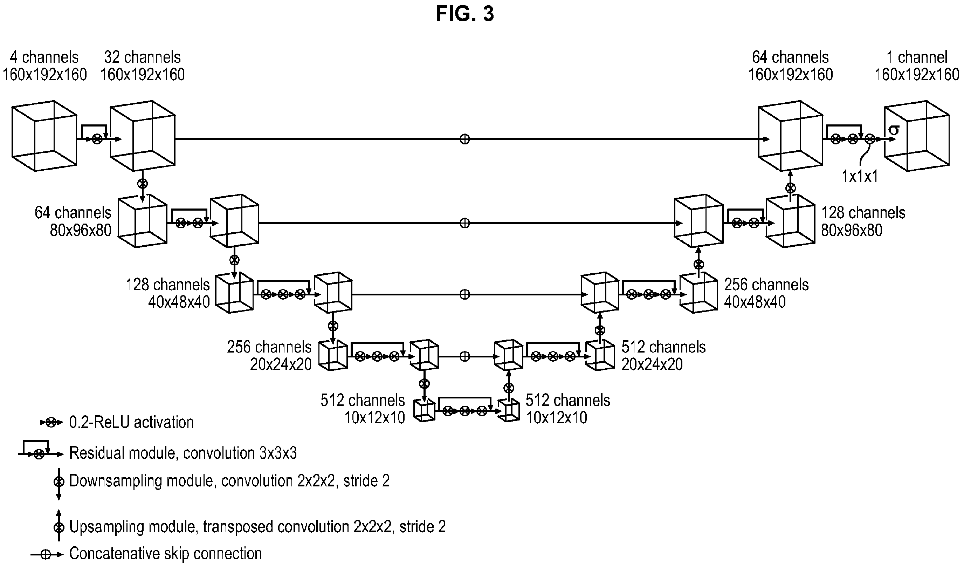

The **figure 3** depicts an exemplary architecture of dose minimization CNN, of the U-Net type. Note that in the case of tridimensional pre-contrast/contrast images, the CNN is itself a tridimensional network as in the example of figure 2, handling quadridimensional feature maps.

U-Net is a neural network of the encoder-decoder type: it comprises an encoder branch (or "contracting path") that maps the input (at least the pre-contrast image and the low dose image/first dose image) into a high-level representation and then a decoder branch (or "expanding path") generating the output image (the standard dose image/second dose image) from the high-level representation.

U-Net further comprises skip (or "lateral") connections between the encoder branch and decoder branch.

The encoder branch, acts as a backbone, and can be seen as a conventional feature extraction network that can be of many types, and in particular a conventional CNN, preferably a fully convolutional neural network (direct succession of blocks of convolution layers and non-linear layers such as ReLU (rectified linear unit), that in particular alternates residual and strided convolution blocks to downsample). The encoder branch extracts from the input image a plurality of initial feature maps representative of the input image at different scales. More precisely, the backbone consists of a plurality of successive convolution blocks, such that the first block produces a first initial feature map from the input, then the second block produces a second initial feature map from the first initial feature map, etc.

It is conventionally understood for convolutional neural networks that the scale is smaller with each successive map (in other words the resolution decreases, the feature map becomes "smaller" and therefore less detailed), but of greater semantic depth, since increasingly high-level structures of the image have been captured. Specifically, initial feature maps have increasing numbers of channels as their spatial size decreases.

In practice, a pooling layer is placed between two blocks to decrease the scale by a factor of 2 (typically 2x2x2 convolution with stride 2 for down sampling in case of 3D images), and from one block to another the number of filters of the convolution layers used (generally $3 \times 3 \times 3$ convolutions) is increased (and preferably doubled).

In the 5-level standard U-Net there is for example successive channel numbers of 32, 64, 128, 256 and 512, and successive map spatial sizes (for a $160 \times 192 \times 160$ input image) of $160 \times 192 \times 160$, $80 \times 96 \times 80$, $40 \times 48 \times 40$, $20 \times 24 \times 20$, $10 \times 12 \times 10$. We see that the input has already 4 channels - 3 pre-contrast images (T1, T2-flair ADC) and 1 contrast image (T1c- in the training and T1c in operation), while the output as a single channel (T1c in the training and T1c+ in operation).

The feature maps obtained by the encoder branch are said to be initial because they will be reprocessed by the decoder branch. Indeed, as explained, "low-level" maps have a higher spatial resolution but a shallow semantic depth. The decoder branch aims to increase their semantic depth by incorporating the information from the "high-level" maps.

Thus, said decoder branch of the CNN has the symmetrical architecture of the encoder branch, as it generates, from the initial feature maps, a plurality of enriched feature maps that are again representative of the input image at different scales, but they incorporate the information from the initial feature maps of smaller or equal scale while reducing the number of channels.

In other words, the decoder branch also consists of a plurality of successive convolution blocks but in opposite order, such that the first block produces the first enriched feature map (from which the output image may be directly generated) from the second enriched feature map and the first initial feature map, after the second block produces the second enriched feature map from the third enriched feature map and the second initial feature map, etc. The decoder branch is also preferably a fully convolutional CNN (direct succession of blocks of convolution layers and non-linear layers such as ReLU, that in particular alternates residual and strided convolution blocks to upsample),

In more details, each i-th enriched map has the scale of the corresponding i-th initial map (i.e. sensibly same spatial size) but incorporates the information of all j-th maps, for each j≥i. In practice, each i-th enriched map Di is generated according to the corresponding i-th initial map Ei and/or the next (i+1-th) enriched map, hence the "contracting and expansive" nature of the branches (i.e. "U" shaped): the initial maps are obtained in ascending order and then the enriched maps are obtained in descending order.

Indeed, the maximum semantic level is obtained at the "smallest-scale" map, and from there each map is enriched on the way back down again with the information of the already enriched maps. The skip connections between the encoder branch and the decoder branch provide the decoder branch with the various initial maps.

Typically, the generation of an enriched map based on the corresponding initial map and the smaller-scale enriched map comprises rescaling of the enriched map, typically doubling the scale (if there has been halving of scale in the encoder branch), i.e. up sampling of the enriched feature map with by a $2 \times 2 \times 2$ convolution ("up-convolution") that halves the number of feature channels, then concatenation with the corresponding initial map Ei (cropped of necessary, both maps are now sensibly the same scale) to double again the number of channels, and from one block to another the number of filters of the convolution layers used (generally $3 \times 3 \times 3$ convolutions) is again decreased (and preferably further halved).

Downsampling and upsampling convolution blocks rely on $2\times2\times2$ kernels, all other kernels are $3\times3\times3$ at the exception of the final $1\times1\times1$ convolution. All activation functions but the last sigmoid are 0.2-LeakyReLU.

Note that the present invention is not limited to the specific U-Net architecture: could be suitable any CNN which is trained to reconstruct, from at least a training pre-contrast image and a training contrast image respectively depicting a body part prior to and after an injection of said low dose of contrast agent, a training contrast image depicting said body part after an injection of said standard dose of contrast agent.

The training will be described below.

*Method for medical imaging*

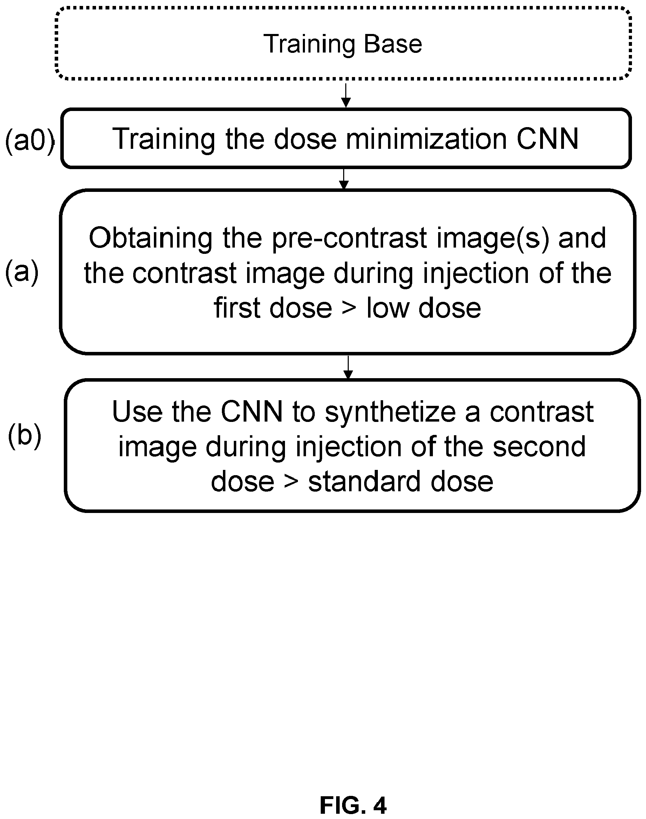

As represented by **figure 4,** the present method starts with a step (a) of obtaining said pre-contrast image and contrast images to be processed (referred to as candidate pre-contrast and contrast images), in particular T1-weighted pre-contrast/contrast images, preferably from a medical imaging device 10 connected to the second server 1b.

The candidate pre-contrast image and candidate contrast image respectively depict a body part prior to and after an injection of a first dose of contrast agent, wherein said first dose is higher than a predetermined low dose, the predetermined reduced dose being lower than a predetermined standard dose.

In the preferred MRI embodiment, step (a) comprises obtaining three candidate pre-contrast images (the candidate T1-weighted pre-contrast image, a candidate T2-flair-weighted pre-contrast image, and a candidate ADC pre-contrast map).

Note that this step (a) may be implemented by the data processor 11b of the second server 1b and/or by the medical imaging device 10.

In a main step (b), implemented by the data processor 11b of the second server 1b, the CNN is applied to the candidate pre-contrast image and the candidate contrast image, so as to generate a synthetic contrast image simulating said body part after an injection of a second dose of contrast agent which is higher than the standard dose.

In the preferred MRI embodiment, the CNN is applied to the three candidate pre-contrast images and the candidate contrast image.

*Training method*

The method advantageously comprises a previous step (a0) of training the CNN, implemented by the data processor 11a of the first server 1a.

By training, it is meant the determination of the optimal values of parameters and weights or the CNN. Note that alternatively the CNN may be directly taken "off the shelf" with preset values of parameters and weights.

Said training method can be performed according to the prior art, and any suitable training protocol known to a skilled person may be used.

It is here to be understood that the CNN is trained to perform its original "dose minimization" task, i.e. to reconstruct, from at least a training pre-contrast image and a training contrast image respectively depicting a body part prior to and after an injection of said low dose of contrast agent, a training contrast image depicting said body part after an injection of said standard dose of contrast agent.

Indeed, it is impossible to directly train a CNN to generate, from at least a training pre-contrast image and a training contrast image respectively depicting a body part prior to and after an injection of a first dose of contrast agent higher than the lower dose, the synthetic contrast image simulating said body part after an injection of a second dose of contrast agent which is higher than the standard dose: such training would require having training images after an injection of the second dose, thereby making the total injected dosage amount greater than the dosage amount suggested by recent clinical guidelines

By contrast, to learn the "dose minimization" task, the training base has just to comprise a plurality of sequences of at least one training pre-contrast image (as explained there could be possibly three pre-contrast images), an "initial" training contrast image and a "final" training contrast image respectively depicting a body part prior to, after an injection of said low dose of contrast agent, and after an injection of said standard dose of contrast agent.

The CNN is trained to predict the final training contrast image (as ground truth) from the training pre-contrast image(s) and the initial contrast image of the same sequence.

In more details, for generating a training example, we perform the normal protocol for acquiring a contrast image depicting a body part after an injection of said standard dose of contrast agent, but the administration of the standard dose is simply split in two successive injections (1) the low dose and (2) the difference between the low dose and the standard dose, for example 0.025mmol/kg and 0.1-0.025=0.075mmol/kg of GBCA as explained, and an additional contrast image is acquired in between as the "initial" contrast image, this image actually depicting the body part after an injection of said low dose of contrast agent. Note that two-injection MRI protocols have been recently included in consensus guidelines for glioma imaging, the first injection playing the role of preload bolus for a possible later perfusion sequence. Consequently, it is harmless.

To sum up, for a patient:

- the pre-contrast image(s) is(are) acquired;
- the low dose is injected and a first contrast image is acquired;
- the difference between the standard dose and the low dose is injected (so that the patient receives in total the standard dose), and a second contrast image is acquired;
- the sequence of the pre-contrast image(s), the two successive contrast images is labeled as a training example.

*Tests*

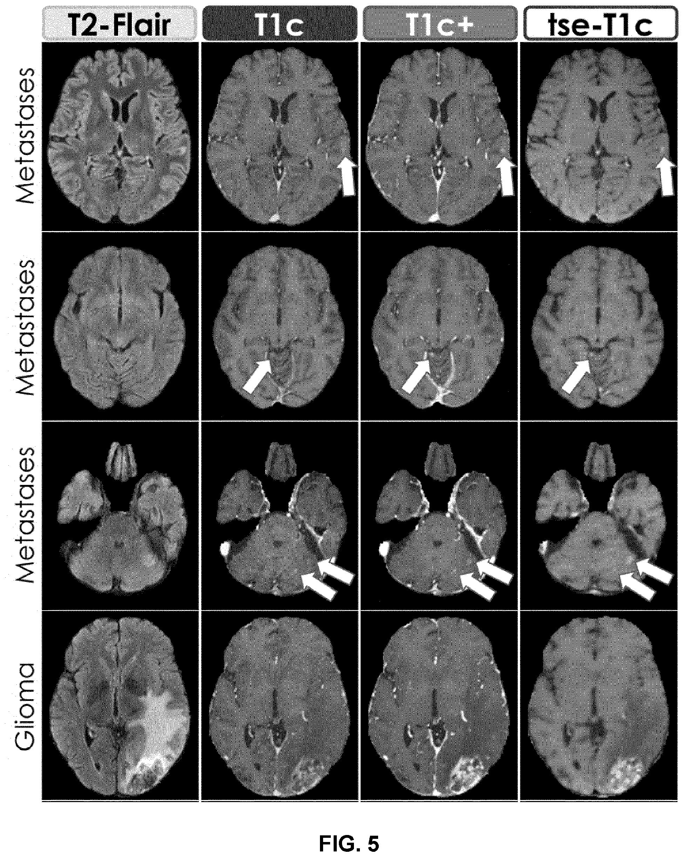

**Figure 5** displays the T2-Flair, T1c, T1c+ and tse-T1c images for four example MRI exams from the test sample, either with brain metastases or glioma. We can see that the T1c+ images (the synthetic super dose contrast images) present an excellent quality and are easier to read.

Table 1 reports average image quality (IQ) scores for the T1c, T1c+ and tse-T1c images from 79 exams of a test sample. Grades are expressed on a 4-point Likert scale ranging from 1 (poor) to 4 (excellent). Standard deviations are given between parentheses. Differences across readers and post-contrast MRI images are compared using two-tailed t-tests, and p-values are reported. Best metrics are emphasized using a bold font when the 5% significance threshold is met)

This table shows the synthetized T1c+ images were preferred by two readers (neuroradiologists with respectively 10 and 11 years of experience) for their general image quality, when no quality difference was found between T1c and tse-T1c images. On average between readers, T1c and tse-T1c were graded 2.7/4 (average to good), when T1c+ were graded 3.4/4 (good to excellent).

Table 1

| | IQ ±std (grades on a 4-point scale) | | | *T1c vs. T1c+* | *T1c vs. tse-T1c* | *T1c+ vs. tse-T1C* |
|---|---|---|---|---|---|---|
| | **T1c** | **T1c+** | **tse-T1c** | | | |
| **Reader Average** | 2.7 (±0.5) | **3.4** (±0.3) | 2.7 (±0.6) | *P<.001** | *P=.62* | *P<.001** |
| **Reader 1** | 2.8 (±0.5) | **2.9** (±0.4) | 2.7 (±0.6) | *P=.02** | *P=.36* | *P=.007** |
| **Reader 2** | 2.6 (±0.7) | **3.9** (±0.4) | 2.7 (±0.8) | *P<.001** | *P=.14* | *P<.001** |
| *R1 vs. R2* | *P=.007** | *P<.001** | *P=.89* | | | |

Table 2 reports the average contrast-to-noise ratio (CNR), lesion-to-brain ratio (LBR), and contrast enhancement percentage (CEP) performance metrics for the T1c, T1c+ and tse-T1c images from 52 exams of the test sample with at least one reference lesion. Standard deviations are given between parentheses. Differences across readers and post-contrast MRI images are compared using two-tailed t-tests, and p-values are reported. Best metrics are emphasized using a bold font when the 5% significance threshold is met.

With an average CNR of 44.5, LBR of 1.66 and CEP of 112.4%, the synthetized T1c+ images outperform both T1c and tse-T1c images for all the considered metrics.

Table 2

| | **T1c** | **T1c+** | **tse-T1c** | *T1c vs. T1c+* | *T1c vs. tse-T1c* | *T1c+ vs. tse-T1C* |
|---|---|---|---|---|---|---|
| **CNR** | 9.1 (±5.8) | **44.5** (±25.7) | 16.8 (±9.7) | *P<.001** | *P<.001** | *P<.001** |
| **LBR** | 1.31 (±0.18) | **1.66** (±0.27) | 1.44 (±0.24) | *P<.001** | *P<.001** | *P<.001** |
| **CEP** | 85.6 (±35.5) | **112.4** (±36.5) | 92.2 (±38.1) | *P<.001** | *P=.0.11* | *P<.001** |

Finally, the average lesion detection performance reached when reading the T1c images, and when also jointly reading its post-processed T1c+ counterpart, was compared. In both reading scenarios, the pre-contrast T2-Flair image was available to readers as well. A read with access to T2-Flair, T1c and tse-T1c images defined 187 reference lesions with a median long axis length of 9.2mm (inter-quartile range of 10.7mm). Three nested evaluation configurations were considered, depending on the minimum included lesion size: 10mm, 5mm or 0mm - all lesions being considered in this case.

The access to T1c+ images increased the lesion detection sensitivity (SE) for both readers in all evaluation configurations. On average across readers, the overall SE was 75% when T1c+ images were available (59% with T1c images only, P<.001*), 85% for lesions larger than 5mm (70% with T1c, P<.001*), and 96% for lesions larger than 10mm (88% with T1c, P=.008*). No difference was found in terms of FDR (false detection rates), which remained below 0.19/exam across readers, reading scenario, and evaluation configurations. No difference was found between readers for neither SE nor FDR.

PPV remained higher than 90% in all configurations. On average across readers, PPV quantitative differences were 3% at maximum between the two reading scenarios. F1 was higher than 90% across readers and readings for lesions larger than 10mm, higher than 80% for lesions larger than 5mm, and higher 70% when all lesions were included. On average across readers, F1 was higher when T1c+ was available to readers by a margin that varied between 4% and 11% according to considered range of lesion sizes.

*Computer program product*

In a second and a third aspect, the invention provides a computer program product comprising code instructions to execute a method (particularly on the data processor 11a, 11b of the first and/or second server 1a, 1b) according to first second aspect of the invention for medical imaging, and storage means readable by computer equipment (memory of the first or second server 1a, 1b) provided with this computer program product.

**Claims**

1. A method for medical imaging, the method being **characterized in that** it comprises the implementation, by a data processor (11b) of a second server (1b), of steps of:

   (a) Obtaining at least a candidate pre-contrast image and a candidate contrast image respectively depicting a body part prior to and after an injection of a first dose of contrast agent, wherein said first dose is higher than a predetermined low dose, the predetermined reduced dose being lower than a predetermined standard dose; a convolutional neural network, (CNN), being trained to reconstruct, from at least a training pre-contrast image and a training contrast image respectively depicting a body part prior to and after an injection of said low dose of contrast agent, a training contrast image depicting said body part after an injection of said standard dose of contrast agent;
   (b) Applying the CNN to the candidate pre-contrast image and the candidate contrast image, so as to generate a synthetic contrast image simulating said body part after an injection of a second dose of contrast agent which is higher than the standard dose.

2. A method according to claim 1, wherein said low dose is between 10 and 50% of the standard dose, preferably around 25%.

3. The method according to one of claims 1 and 2, wherein said first dose is at least 50% of the standard dose, preferably around 100% of the standard dose.

4. The method according to claims 2 and 3 in combination, wherein said low dose is between 1/5 and 1/3 of the standard dose, preferably around 25%; and said first dose is at least 80% of the standard dose, preferably around 100% of the standard dose; so that said second dose is at least 240% of the standard dose, preferably between 320% and 400% of the standard dose.

5. The method according to one of claims 1 to 4, wherein the candidate pre-contrast image and the candidate contrast image are acquired by a medical imaging device (10) connected to the second server (1b), in particular an MRI scanner.

6. The method according to one of claims 1 to 5, wherein said at least one pre-contrast image and contrast image are T1-weighted images.

7. The method according to claim 6, wherein step (a) comprises obtaining three candidate pre-contrast images, including the T1-weighted pre-contrast image, a T2-flair-weighted pre-contrast image, and an ADC pre-contrast map; the CNN being trained to reconstruct, from a training T1-weighted pre-contrast image, a training T2-flair-weighted pre-contrast image, and a training ADC pre-contrast map and a training T1-weighted contrast image respectively depicting a body part prior to and after an injection of said low dose of contrast agent, a training T1-weighted contrast image depicting said body part after an injection of said standard dose of contrast agent; step (b) comprising applying the CNN to the three candidate pre-contrast image and the candidate contrast image, so as to generate a synthetic T1-weighted contrast image simulating said body part after an injection of a second dose of contrast agent which is higher than the standard dose.

8. The method according to one of claims 1 to 7, wherein said CNN comprises an encoder branch and then a decoder branch, with skip connections between the encoder branch and decoder branch.

9. The method according to one of claims 1 to 8, comprising a previous step of training, by a data processor (11a) of a first server (1a), said CNN from a base of sequences of a training pre-contrast image, a first training contrast image and a second training image respectively depicting a body part prior to, after an injection of said low dose of contrast agent, and after an injection of said standard dose of contrast agent.

10. Computer program product comprising code instructions to execute a method according to one of claims 1 to 9 for medical imaging, when said program is executed on a computer.

11. A computer-readable medium, on which is stored a computer program product comprising code instructions for executing a method according to any one of claims 1 to 9 for medical imaging.

**FIG. 1**

**FIG. 2**

**FIG. 3**

```
                    ┌ ┄ ┄ ┄ ┄ ┄ ┄ ┄ ┄ ┄ ┄ ┄ ┄ ┄ ┄ ┄ ┐
                    ┊          Training Base              ┊
                    └ ┄ ┄ ┄ ┄ ┄ ┄ ┄ ┄ ┄ ┄ ┄ ┄ ┄ ┄ ┄ ┘
```

(a0) | Training the dose minimization CNN

(a) | Obtaining the pre-contrast image(s) and the contrast image during injection of the first dose > low dose

(b) | Use the CNN to synthetize a contrast image during injection of the second dose > standard dose

**FIG. 4**

FIG. 5

Europäisches
Patentamt

European
Patent Office

Office européen
des brevets

## EUROPEAN SEARCH REPORT

**Application Number**

EP 21 30 6909

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BONE ALEXANDRE ET AL: "Contrast-Enhanced Brain MRI Synthesis With Deep Learning: Key Input Modalities and Asymptotic Performance", 2021 IEEE 18TH INTERNATIONAL SYMPOSIUM ON BIOMEDICAL IMAGING (ISBI), IEEE, 13 April 2021 (2021-04-13), pages 1159-1163, XP033917809, DOI: 10.1109/ISBI48211.2021.9434029 [retrieved on 2021-05-17] * abstract * * page 1160, column 1, line 5 - line 7 * * page 1160, column 1, line 21 - line 26 * * page 1160, column 2, line 9 - page 1161, column 1, line 9 * * figure 2 * | 1-11 | INV. G06V10/774 G06V10/82 A61B6/00 G16H30/40 G16H50/50 G16H50/70 G01R33/56 |
| A | WO 2021/061710 A1 (SUBTLE MEDICAL INC [US]) 1 April 2021 (2021-04-01) * paragraph [0047] * * paragraph [0060] * | 1-11 | |
| A | Cupitt Philip: "Patenting Artificial Intelligence at the European Patent Office", , 11 April 2019 (2019-04-11), pages 1-6, XP055928134, Retrieved from the Internet: URL:https://www.marks-clerk.com/insights/patenting-artificial-intelligence-at-the-european-patent-office/ [retrieved on 2022-06-06] * page 5, line 14 - line 19 * | 9 | **TECHNICAL FIELDS SEARCHED (IPC)** G06V G16H A61B G01R |

The present search report has been drawn up for all claims

| Place of search | Date of completion of the search | Examiner |
|---|---|---|
| The Hague | 7 June 2022 | De Coi, Juri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                             

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6909

This annex lists the patent family members relating to the patent documents cited in  the above-mentioned European search report.
The members are as contained in the European Patent Office EDP file on
The European Patent Office is in no way liable for these particulars which are merely given for the purpose of information.

07-06-2022

| Patent document cited in search report | Publication date | Patent family member(s) | Publication date |
|---|---|---|---|
| WO 2021061710    A1 | 01-04-2021 | NONE | |

**EP 4 202 855 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019074938 A

**Non-patent literature cited in the description**

- Can Deep Learning Replace Gadolinium in Neuro-Oncology?. **AMMARI S ; BÔNE A ; BALLEYGUIER C et al.** Invest Radiol. Publish Ah, 2021, 1-9